# EUROPEAN PATENT SPECIFICATION

(11) **EP 3 280 760 B1**
(45) Date of publication and mention of the grant of the patent: **06.10.2021**
(21) Application number: 16741603.1
(22) Date of filing: 19.07.2016
(51) Int. Cl.: C08G 73/02, A01N 33/12, A61L 12/14

(54) **CATIONIC COMPOUNDS AND THEIR USE AS ANTIMYCOTIC AND ANTIMICROBIAL AGENTS**
POLYKATIONISCHE VERBINDUNGEN UND DEREN VERWENDUNG ALS ANTIMYKOTISCHE UND ANTIMIKROBIELLE MITTEL
COMPOSÉS POLYCATIONIQUES ET LEUR UTILISATION COMME AGENTS ANTIMICROBIENS ET ANTIFONGIQUES

(30) Priority: 25.08.2015 EP 15182310
(43) Date of publication of application: 14.02.2018
(73) Proprietor: Visufarma S.p.A., 00136 Roma (IT)
(72) Inventor: REICHL, Stephan, 38518 Gifhorn (DE); VON DEYLEN, Dörte, 38106 Braunschweig (DE); DREHER, Christina, 01307 Dresden (DE); LESSMANN, Frank, 04229 Leipzig (DE); SEIDELMANN, Oliver, 06749 Bitterfeld (DE)
(74) Representative: Reitstötter Kinzebach
(86) International application number: PCT/EP2016/067101
(87) International publication number: WO 2017/032509

(56) References cited:
- DE-T2- 69 013 787
- JP-B2- 3 476 854
- JP-B2- 3 476 854
- US-A- 5 512 597
- US-A1- 2006 002 887
- SHYAM K. SINGH ET AL: "Abstract", ZEITSCHRIFT FUR NATURFORSCHUNG, TEIL B:ANORGANISCHE CHEMIE, ORGANISCHE CHEMIE., vol. 43, no. 6, 1 January 1988 (1988-01-01), pages 778-784, XP055241450, DE ISSN: 0932-0776, DOI: 10.1515/znb-1988-0623
- We C J Ross: "ARYL-2-HALOGENOALKYLARYLAMINES-XXI.* THE DESIGN OF AGENTS TO EXPLOIT DIFFERENCES IN CELLULAR OXIDATION-REDUCTION POTENTIALS", Biochemical Pharmacology, 1 January 1964 (1964-01-01), pages 969-982, XP055306715, Retrieved from the Internet: URL:http://www.sciencedirect.com/science/a rticle/pii/0006295264900930
- JOANNE MCGEACHIE ET AL: "Abstract", ZEITSCHRIFT FUR NATURFORSCHUNG- SECTION B JOURNAL OF CHEMICAL SCIENCES, vol. 41, no. 10, 1 January 1986 (1986-01-01), pages 1255-1259, XP055306721, ISSN: 0932-0776, DOI: 10.1515/znb-1986-1011

## Description

The invention relates to cationic compounds and their use as antimycotic and antimicrobial agents, in particular as disinfectants and preservatives in ophthalmic preparations.

Cosmetics and drugs for multiple use are in general subject to microbial deterioration. In order to ensure the microbial quality of such products during storage and use preservatives are added to the products. The regulatory requirements for marketing authorization and use of preservatives become more and more stringent.

At least a substantial number of the approved preservatives show significant side-effects. Due to the sensitivity of the eye this is especially critical in the ophthalmic field.

One preservative that is mainly used in ophthalmics is benzalkonium chloride (BAC). It exhibits a broad activity spectrum (also against fungi and yeasts) in a wide pH range with a low allergenic potential. However, BAC interferes with the tear film of the eye what may result in the dry eye symptom. In addition, BAC may have cytotoxic effects.

Alternative preservatives, such as Polyquad, a polycationic polymer, which is first described in US 3,931,319 and sodium chlorite are less toxic than BAC but have a diminished activity spectrum, in particular against fungi including yeasts. Therefore, a combination with boric acid or boric acid salts is used in commercial products at the expense of compatibility (non-toxicity).

Also other polycationic compounds or polymers are used as antimicrobial agents. For example, EP 676 437, WO 02/080939 and WO 2004/046109 (US 2006/002887) disclose piperidinium ionenes and pyridinium ionenes for the treatment of microbial infections and for disinfection of medical devices, implants and the like. DE 19646726, EP 1 050 304, US 5,512,597, WO 90/09405, WO 91/09523 and WO 2013/138820 describe polymeric, quarternary ammonium salts which are useful as preservatives or disinfectants for ophthalmic devices, such as contact lenses. According to DE 2930865, polymers having quaternary ammonium groups are used as disinfectants. WO 2013/064798 describes the use of polycationic polymers in wood preservative formulations. Z. Naturforsch. 39b, 74-78 (1984) and 43b, 778-784 (1988) disclose the preparation of 1,1"-alkanediyl-bis-3,3'- and -4,4'-bipyridine salts and their physico-chemical properties. US 2006/002887 A1 describes low molecular weight ionene oligomers useful as antimicrobial substances. DE 690 13 787 T2 describes quaternary ammonium compounds for use in fungicidal and bactericidal compositions. US 5,512,597 A describes polymeric quaternary ammonium compounds and their use as ophthalmic antimicrobials. JP 3 476854 B2 describes polycationic polymers for use as bactericidal and algicidal agents. Singh et al., "Synthesis of Quaternary Salts from 3,3'-Bipyridine and Dibromoalkanes", Z. Naturforsch., 43b, 1987, p. 778 to 784, describe the synthesis of quaternary bipyridine derivatives. Ross, "Aryl-2-halogenoalkylarylamines-XXI.: The design of agents to exploit differences in cellular oxidation-reduction potentials", Biochem. Pharmacol., 1964, vol. 13, issue 7, p. 969 to 982, describes 2-halogenoethylamino-derivatives for use in the treatment of cancer. McGeachie et al., "Formation of Triradical Trications from Hexaquaternary Salts Derived from 4,4'-Bipyridine", Z. Naturforsch. 1986, 41b, p. 1255 to 1259, describe the synthesis of hexaquaternary salts derived from 4,4'-bipyridine.

The agents known from the prior art have the disadvantage that their activity spectrum and/or compatibility is not fully satisfying. The problem underlying the invention is therefore to provide further agents that exhibit a broad activity spectrum against bacteria and fungi and are of acceptable compatibility so that they can also be used in ophthalmic formulations.

### Summary of the invention

This problem is solved by cationic compounds of formula (I) 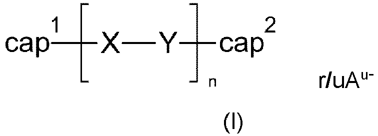 wherein
a)
   X is 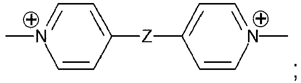
   Y is C₄-C₆ alkenylene;
   Z is C₁-C₆ alkylene;
   cap¹ is 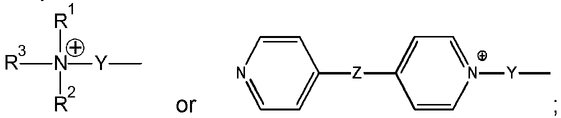
   cap² is 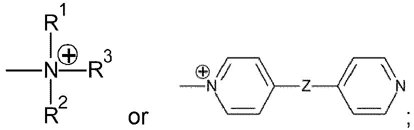
   R¹, R² and R³ are independently of each other C₁-C₆ alkyl or C₁-C₆ hydroxyalkyl or one of R¹, R² and R³ is C₈-C₁₈ alkyl and the other two are independently of each other C₁-C₆ alkyl or C₁-C₆ hydroxyalkyl; wherein R¹, R² and R³ are preferably C₁-C₆-alkyl;
   and
   n is 1 to 250;
b)
   X is 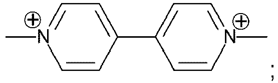
   Y is C₂-C₆ alkylene;
   cap¹ is 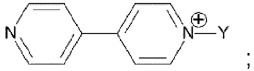
   cap² is 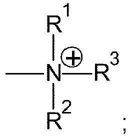
   R¹ and R² are independently of each other C₁-C₆ alkyl;
   R³ is C₈-C₁₈ alkyl; and
   n is 0 to 2;
c)
   X is 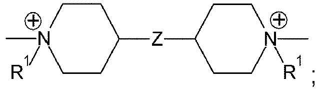
   Y is C₂-C₆ alkylene;
   Z is C₁-C₆ alkylene which is different from Y;
   cap¹ is 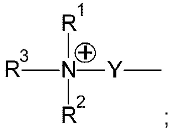
   cap² is 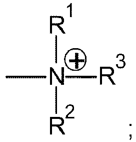
   R¹, R² and R³ are independently of each other C₁-C₆ alkyl; and
   n is 5 to 200, preferably 30 to 150;
d)
   X is 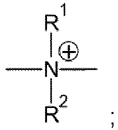
   Y is C₂-C₆ alkylene;
   cap¹ is 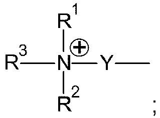
   cap² is 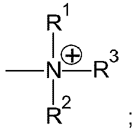
   R¹ and R² are independently of each other C₁-C₆ alkyl;
   R³ is C₁₂-C₁₆ alkyl; and
   n is 100 to 400;
A is an anion;
u is the valency of the anion; and
r is the number of positive charges in the compound (number of positive charges in group (a): 2n+2; in group (b): 2n+2; in group (c): 2n+2; in group (d): n+2).

Preferred compounds of formula (I), group (a) correspond to the formula (Ia): 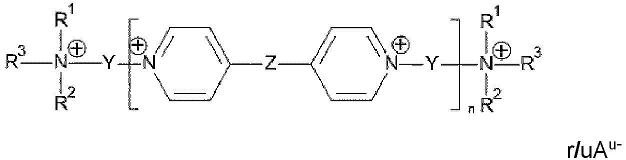 wherein n, r, u, A, Y, Z, R¹, R² and R³ are as defined above.

In formula (Ia), R¹, R² and R³ are preferably methyl or ethyl.

In formula (Ia), Y is preferably C₄-alkenylene.

In formula (Ia), Z is preferably C₂-C₄ alkylene.

In formula (Ia), n is preferably 170 to 220.

Preferred compounds of formula (I), group (b) correspond to formula (Ib): 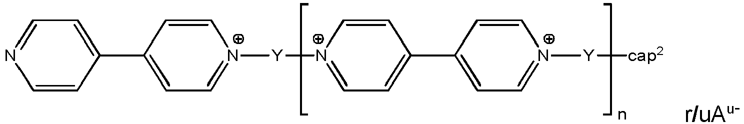 wherein n, r, u, A, Y and cap² are as defined above.

In formula (lb), cap² is preferably 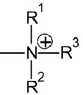 and R¹ and R² are preferably methyl.

In formula (lb), R³ is preferably C₁₀-C₁₆ alkyl, in particular C₁₀-C₁₂ alkyl and most preferably C₁₀ alkyl or C₁₁ alkyl.

In formula (lb), Y is preferably C₂-C₄ alkylene.

In formula (lb), n is preferably 0.

Preferred compounds of formula (I), group (c) correspond to formula (Ic): 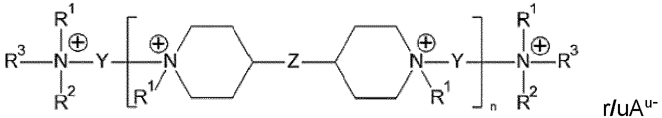 wherein n, r, u, A, Y, R¹, R² and R³ are as defined above.

In formula (Ic), R¹, R² and R³ are preferably methyl.

In formula (Ic), Z is preferably C₁-C₃ alkylene.

In formula (Ic), Y is preferably C₄-C₆ alkylene.

In formula (Ic), n is preferably 90 to 140.

Preferred compounds of formula (I), group (d) correspond to formula (Id): 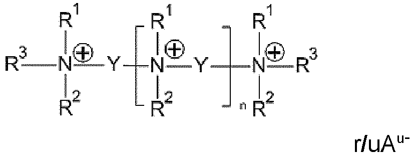 wherein n, r, u, A, Y, R¹, R² and R³ are as defined above.

In formula (Id), R¹ and R² are preferably methyl.

In formula (Id), Y is preferably C₃-C₄ alkylene.

In formula (Id), n is preferably 90 to 140.

Preferably, the cationic compounds of formula (I) are selected from 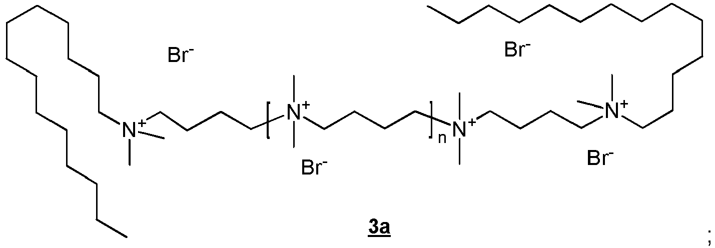 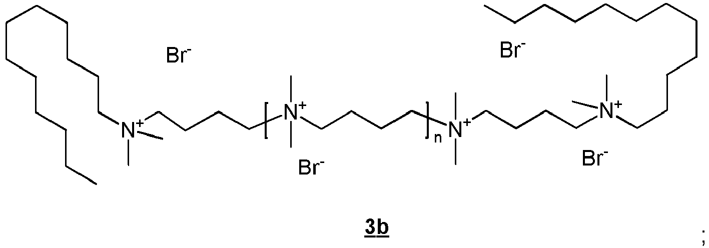 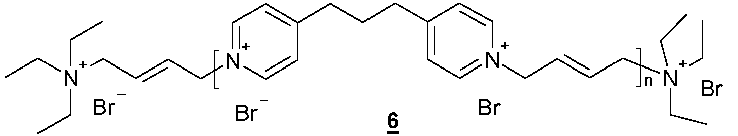 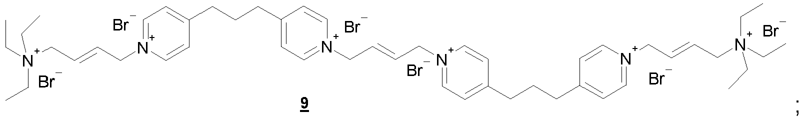 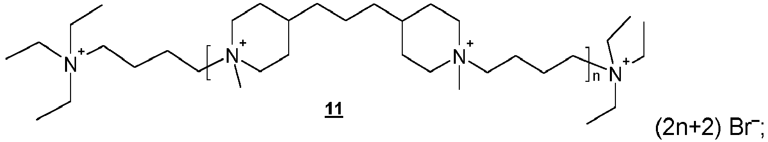 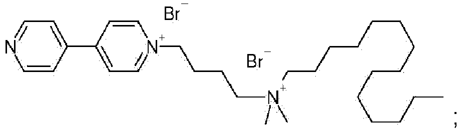 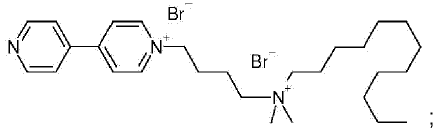 and

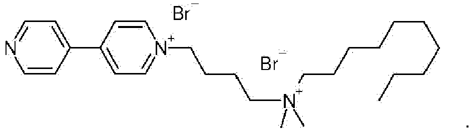

Further provided is a composition comprising a cationic compound of formula (I) as described above.

The composition may be in the form of a disinfectant formulation for medical devices, in particular contact lenses.

The composition may also be in the form of a preservative formulation for liquid, semi-solid or solid drugs, ophthalmic formulations or cosmetics, preferably in the form of an ophthalmic composition.

The composition preferably comprises 0.01 to 1.0 wt.-% of the cationic compound of formula (I).

The composition preferably comprises an additional disinfectant or preservative.

Further provided is the use of a cationic compound of formula (I') as a disinfectant or preservative 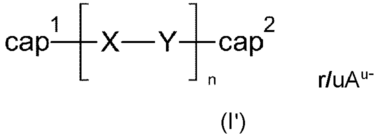 wherein
a)
   X is 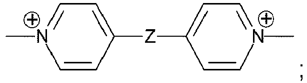
   Y is C₄-C₆ alkenylene;
   Z is C₁-C₆ alkylene;
   cap¹ is 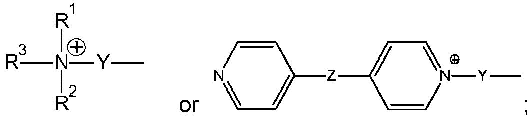
   cap² is 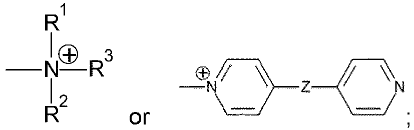
   R¹, R² and R³ are independently of each other C₁-C₆ alkyl or C₁-C₆ hydroxyalkyl or one of R¹, R² and R³ is C₈-C₁₈ alkyl and the other two are independently of each other C₁-C₆ alkyl or C₁-C₆ hydroxyalkyl; and
   n is 1 to 250;
b)
   X is 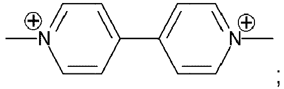
   Y is C₂-C₆ alkylene;
   cap¹ is 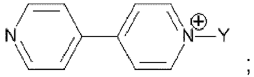
   cap² is 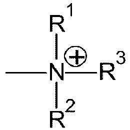 or a halogen atom;
   R¹ and R² are independently of each other C₁-C₆ alkyl;
   R³ is C₈-C₁₈ alkyl; and
   n is 0 to 2;
c)
   X is 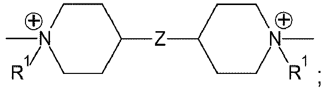
   Y is C₂-C₆ alkylene;
   Z is C₁-C₆ alkylene which is different from Y;
   cap¹ is 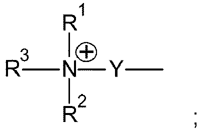
   cap² is 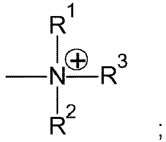
   R¹, R² and R³ are independently of each other C₁-C₆ alkyl; and
   n is 5 to 200, preferably 30 to 150;
d)
   X is 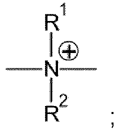
   Y is C₂-C₆ alkylene;
   cap¹ is 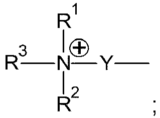
   cap² is 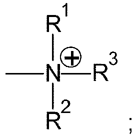
   R¹ and R² are independently of each other C₁-C₆ alkyl;
   R³ is C₁₂-C₁₆ alkyl; and
   n is 100 to 400;
A is an anion;
u is the valency of the anion; and
r is the number of positive charges in the compound.

Further provided is the use of a cationic compound of formula (I) or of a composition as described above as a disinfectant or preservative.

Further disclosed herein is a method of disinfecting or preserving liquid, semi-solid or solid drug formulations, medical devices, in particular contact lenses, or cosmetics which comprises adding a cationic compound as described above or of a composition as described above to said drug formulations or cosmetics or contacting said medical devices, in particular contact lenses, with a cationic compound as described above of a composition as described above.

Further disclosed herein is the use of a cationic compound as described above or of a composition as described above as an antimycotic agent.

Further disclosed herein is a cationic compound as described above or of a composition as described above for use as an antimycotic agent.

Further disclosed herein is the use of a cationic compound as described above or of a composition as described above as an antibacterial agent.

Further disclosed herein is a cationic compound as described above or of a composition as described above for use as an antibacterial agent.

### Brief description of the drawings:

Fig. 1 shows the course of the TEER value for the compound of example 2
Fig. 2 shows the course of the TEER value for the compound of example 3
Fig. 3 shows the course of the TEER value for the compound of comparative example 1
Fig. 4 shows the course of the TEER value for the compound of comparative example 2
Fig. 5 shows the course of the TEER value for the compound of reference example 5
Fig. 6 shows the course of the TEER value for the compound of example 4
Fig. 7 shows the course of the TEER value for the compound of example 1.2

### Detailed description of the invention

The term "alkyl" as used herein means a straight or branched alkyl group having a number of carbon atoms as indicated. Examples for "alkyl" are methyl, ethyl, n-propyl, isopropyl, n-butyl, isobutyl, t-butyl, n-pentyl or n-hexyl. Further examples are n-nonyl, isononyl, n-decyl, n-dodecyl, n-tridecyl, isotridecyl, n-tetradecyl, n-hexadecyl and n-octadecyl.

The term "alkylene" as used herein means a saturated, straight or branched hydrocarbon group derived by the removal of two hydrogen atoms from the same or two different carbon atoms of a parent alkane. The number of carbon atoms is as indicated. Examples for "alkylene" are methylene (-CH₂-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), isobutylene (-CH(CH₃)CH₂CH₂-), 2-methylpropylene (-CH₂CH(CH₃)CH₂-), n-pentylene (-CH₂CH₂CH₂CH₂CH₂-) or n-hexylene (-CH₂CH₂CH₂CH₂CH₂CH₂-).

The term "alkenylene" as used herein means an unsaturated, straight or branched hydrocarbon group derived by the removal of two hydrogen atoms from the same or two different carbon atoms of a parent alkene. An alkenylene group as used herein has four to six carbon atoms and one double bond. The double bond is not terminal and the term includes E and Z isomers. Examples for "alkenylene" are 2-butenylene (-CH₂CH=CHCH₂-), 2-methyl-2-butenylene (-CH₂C(CH₃)=CHCH₂-), 2-pentenylene (-CH₂CH=CHCH₂CH₂-), 2-methyl-2-pentenylene (-CH₂C(CH₃)=CHCH₂CH₂-) or 2-methyl-3-pentenylene (-CH₂CH(CH₃)CH=CHCH₂-).

Examples for the term "anion" are inorganic anions such as Cl⁻, Br⁻, I⁻, SO₄²⁻, HSO₄⁻, CO₃²⁻, HCO₃⁻, PO₄³⁻, HPO₄²⁻, or H₂PO₄⁻ or organic ions derived from carboxylic or sulfonic acids such as acetate, sorbate or methylsulfonate. Br is preferred.

The cationic compounds of the invention can be prepared as follows:
Compounds of group (a):
   A α,ω-alkylenedipyridine compound is reacted under heating with a α,ω-dihalogeno-C₄-C₆-alkene in an aprotic or protic polar solvent, such as ketones like acetone; acetonitrile;
   ethylacetate; C₁-C₄alkanols like methanol, ethanol, n-propanol, isopropanol, n-butanol,
   isobutanol or t-butanol; dimethylformamide or water or mixtures thereof, and in the presence of a tri-C₁-C₆ alkylamine. The α,ω-dihalogenO-C₄-C₆-alkene is preferably a α,ω-dichloro or
   α,ω-dibromo-C₄-C₆-alkene.
Compounds of group (b):
   4,4'-bipyridine is reacted under heating with an α,ω-dihalogeno-C₁-C₆-alkane in an aprotic or
   protic polar solvent, as given under (a) above , and in the presence of a di-C₁-C₆-alkyl-C₈-C₁₈-alkylamine. As dihalogeno-C₁-C₆-alkane a dichloro or dibromo-C₁-C₆-alkane is used.
Compounds of group (c):
   A α,ω-C₂-C₆-alkylene-N,N'-di-C₁-C₆-alkyldipiperidine is reacted under heating with an α,ω-dihalogeno-C₁-C₆-alkane in an aprotic or protic polar solvent, as given under (a) above , and
   in the presence of a tri-C₁-C₆-alkylamine. As dihalogeno-C₁-C₆-alkane a dichloro or dibromo-C₁-C₆-alkane is used.
Compounds of group (d):
   A N,N,N',N'-tetra-C₁-C₆-alkyl-α,ω-C₁-C₆-alkane diamine is reacted under heating with an α,ω-dihalogeno-C₁-C₆-alkane in an aprotic or protic polar solvent, as given under (a) above, and
   in the presence of a di-C₁-C₆-alkyl-C₉-C₁₈-alkylamine. As dihalogenoalkane a dichloro or dibromo-C₁-C₆-alkane is used.

The compounds of the invention are cationic, including polycationic, compounds having antimicrobial and antimycotic activity against bacteria and, in particular, fungi including yeasts. It was most surprising that the compounds of the invention have also activity against aspergillus brasiliensis (aspergillus niger). Furthermore, the compounds of the invention are highly compatible with sensitive tissues and non-irritating. The compounds of the invention are therefore useful as preservatives of formulations (compositions) that are subject to deterioration by bacteria, yeasts or fungi. Such formulations are in particular those for multiple use such as liquid drug formulations such as aqueous or non-aqueous solutions or suspensions, semi-solid drug formulations for topical treatment such as creams or ointments, solutions for disinfecting medical devices, or cosmetics. Preferably, the compounds of the invention are used in ophthalmic formulations such as solutions that are directly applied to or in contact with ocular tissues or solutions for treating ophthalmic devices such as contact lenses or other ophthalmic devices. Such solutions include, for example, eye drops, eyewash solutions, and solutions for contact lens care such as cleaning solutions or storing solutions, and solutions for disinfecting other ophthalmic devices.

The compounds of the invention are further useful as active compounds in other medicinal formulations such as ophthalmic solutions for treating the eye, nose drops, or antimycotic compositions or in disinfecting formulations.

The compounds of the invention are also useful as preservative for wood, leather or food products as well as for cosmetics such as shampoos, hand and body lotions, moisturizing and cleansing emulsions, antiperspirants, and deodorants.

The present compositions or formulations comprising the compounds of the invention can be formulated, for example, as disinfecting compositions, cleaning compositions, wetting compositions, conditioning compositions, soaking compositions and, in particular, ophthalmic compositions. Also, the present compositions can be formulated to be useful in performing two or more contact lens caring operations such as a disinfecting/cleaning composition, or a cleaning/conditioning composition or even an all purpose lens care composition.

The compounds of the present invention are usually contained in a formulation or composition of the invention at a concentration ranging from 0.0001 to 1.0 w/v%, preferably from 0.001 to 0.1 w/v%, weight per volume of the formulation or composition. For example, disinfectants for ophthalmic devices are formulated using the compounds usually at a concentration ranging from 0.0001 to 1.0 w/v%, preferably from 0.001 to 0.1 w/v%. Eye drops, eyewash solutions, or cleaning solutions, storing solutions, or cleaning-storing solutions used for contact lens care are formulated using the compounds usually at a concentration ranging from 0.0001 to 0.01 w/v%, preferably from 0.0005 to 0.005 w/v%.

There is no restriction on the pH of the compositions or formulations of the present invention as long as the pH is within the physiologically, in particular ophthalmologically acceptable range; the pH value usually ranges from about 5.0 to 9.0, preferably from 5.5 to 8.5. The osmotic pressure ratio of ophthalmic formulations (the ratio of osmotic pressure of the ophthalmic solution to the osmotic pressure of physiological saline) is usually adjusted to about 0.5 to 5.0, preferably to about 0.8 to 2.0.

The ophthalmic solutions of the present invention can be formulated with various ingredients besides the compounds of the invention. There are no restrictions on the ingredients contained in the solutions. For example, the formulations may be formulated with a variety of additives such as buffering agents, isotonizing agents, solubilizers, stabilizers, viscoelastic agents, chelating agents, and pH-adjusting agents as well as active ingredients such as agents for removing congestion, anti-inflammatory agents, astringents, antihistaminic agents, anti-microbial agents, glaucomatosa, steroids, vitamins, amino acids, inorganic salts, and saccharides. The buffering agents include, for example, borate buffer, phosphate buffer, carbonate buffer, acetate buffer, citrate buffer, ε-aminocapronic acid, glutamic acid and salts thereof, and aspartic acid and salts thereof. The isotonizing agents include, for example, sodium chloride, potassium chloride, calcium chloride, glycerol, glucose, mannitol, aminoethyl sulfonic acid, aspartic acid, potassium aspartate, sodium aspartate, and magnesium potassium aspartate. In particular, the preferable isotonizing agents are aspartic acid and/or salts thereof; the salts preferred are sodium aspartate, potassium aspartate, and magnesium aspartate. The ophthalmic solutions are formulated with a solution of aspartic acid and/or salts thereof that is isotonic with 0.5 to 2.0% sodium chloride solution.

The present compositions may include other, e.g., complementary and/or potentiating, antimicrobial agents. Examples of such other antimicrobial agents include thimerosal, sorbic acid, 1.5-pentanedial, alkyl triethanolamines, boric acid, other polycationic compounds such as benzalconium chloride, physiologically acceptable salts of any of the above, 3-chloroallyl-3,5,7-triaza-1-azoniaadamantine chloride, phenylmercuric salts and mixtures thereof.

Thus, the present compositions can be formulated, for example, as disinfecting compositions, cleaning compositions, wetting compositions, conditioning compositions, soaking compositions. Also, the present compositions can be formulated to be useful in performing two or more contact lens caring operations such as a disinfecting/cleaning composition, or a cleaning/conditioning composition or even an all purpose lens care composition.

The following examples illustrate the invention without limiting it.

The molar mass M_{w} of polymers where given was determined by GPC (gel permeation chromatography) as follows. Suitable columns are those charged with polyacrylate/methacrylate beads that are surface-modified by NH-functionalization such as columns of the Novema Max series which are commercially available from PSS Polymer Standards Service GmbH, Mainz, Germany. Two columns (Novema Max, 10 µm and 30 Å or 1000 Å) were used in series and eluted as described below.

### Measuring conditions:

Solvent: buffer A (0.1% trifluoro acetic acid, 0.05 % NaN₃ in water) and buffer B (acetonitrile) in a ratio of 90:10 v/v.
Flow: 1 ml/min at about 80 bar.
Detection: RID
Concentration: 5 g/l
Injection volume: 100 µl
Calibration: pullulan available from PSS (10 standards with molar peaks from 342 Da to 708000 Da).

### Example 1

### Synthesis of polycationic compound (3a) and (3b)

### 1.1 Polycationic compound (3a)

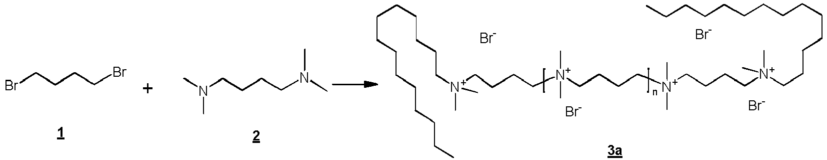

A 100 ml flask equipped with reflux condenser, magnetic stirrer and adsorber tube was charged with 1.26 ml N,N,N',N'-tetramethyl-1,4-butane diamine (1.0 g, 6.9 mmol) in 40 ml acetone (HPLC grade). Then, 1.2 ml N,N-dimethyltetradecylamine (0.96 g, 4.0 mmol, 0.57 equiv.) and 0.91 ml 1,4-dibromobutane (1.65 g, 7.6 mmol, 1.1 equiv.) were added successively. The reaction mixture was stirred under reflux for 15 hours. Thereby a white precipitate was formed. The cooled suspension was filtered off rapidly, the filtrate was washed with 40 ml acetone (HPLC grade) and dried under vacuum at 50 °C for 30 minutes. Compound **3a** was obtained as a white solid (2.85 g, 95% yield). ¹H-NMR (D2O, 500 MHz): δ (in ppm) = 0.84-0.94 (br, alkyl end cap), 1.25-1.45 (br, alkyl end cap), 1.73-1.84 (br, alkyl end cap), 1.85-195 (br, 4H), 3.08-3.18 (br, 6H), 3.40-3.48 (br, 4H); M_{w} ∼42000 g·mol⁻¹ according to the GPC method.

### 1.2 Polycationic compound (3b)

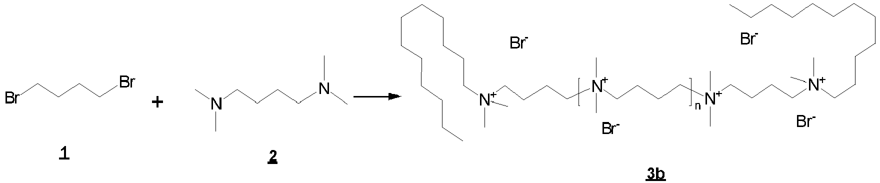

The above prodedure under 1.1 was repeated using 4.0 mmol of N,N-dimethyl-dodecylamine in place of N,N-dimethyltetradecylamine to obtain compound 3b as a white solid.

### Example 2

### Synthesis of polycationic compound (6)

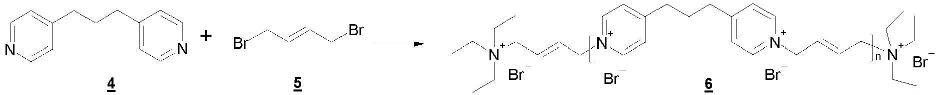

A 100 ml flask equipped with reflux condenser, magnetic stirrer and adsorber tube and charged with a solution of 2.0 g 4,4'-trimethylenedipyridine (10.1 mmol, 1.0 equiv.) in 40 ml acetone (HPLC). Then, 2.37 g trans-1,4-dibromo-2-butene (11.1 mmol, 1.1 equiv.) were added in one portion and the reaction mixture was stirred under reflux for 30 minutes. Subsequently, 0.85 ml triethylamine (0.61 g, 6.1 mmol, 0.6 equiv.) were added and the mixture was stirred under reflux for additional 5 hours. A white precipitation was formed which was filtered off rapidly, washed with 50 ml acetone (HPLC) and dried in vacuum at 50 °C for 30 minutes.

Compound **6** was obtained as a greenish powder (3.12 g, 70% yield). ¹H-NMR (D2O, 500 MHz): δ (in ppm) = 1.32 (t, CH3), 2.20 (m, 2H, CH2), 3.06 (m, 4H, CH2), 3.32 (q, CH2), 5.28 (d, 4H, CH2), 6.28 (m, 2H, CH), 7.98 (d, 4H, Ar-H), 8.72 (d, 4H, Ar-H); M_{w} ∼91.000 g·mol⁻¹ according to the GPC method.

### Comparative examples 1 and 2

In an analogous manner to example 2 the following compounds were prepared:

### Comparative example 1:

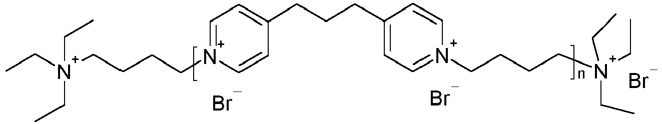

M_{w} ∼75.000 g·mol⁻¹ according to the GPC method.

### Comparative example 2:

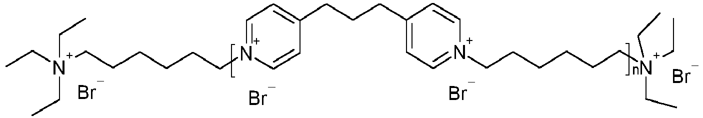

M_{w} ∼82.000 g·mol⁻¹ according to the GPC method.

### Example 3

### Synthesis of the dimeric compound (9)

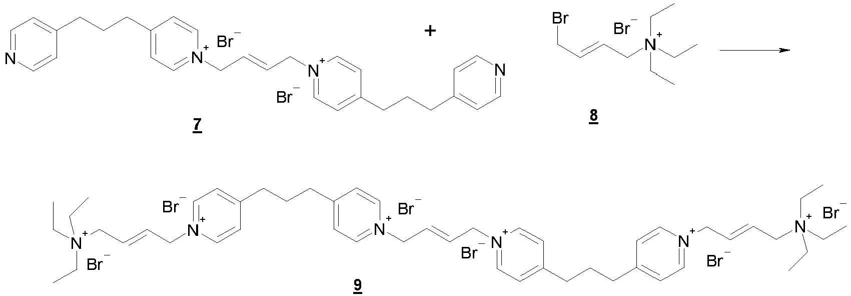

### Synthesis of the centerpiece substructure (7)

A 100 ml flask equipped with a magnetic stirrer was charged with 4.63 g 4,4'-trimethylenedipyridine (23.4 mmol, 10.0 equiv.) in 30 ml acetone (HPLC). Then, 0.5 g trans-1,4-dibromo-2-butene (99%, 2.3 mmol, 1.0 equiv.) were added at once and the clear solution was stirred at 20 °C overnight. Thereby the solution became turbid. The precipitate was filtered off, washed with 20 ml acetone and dried in vacuum at 50 °C. The compound **7** was obtained as a green solid (1.43 g, quantitative). ¹H-NMR (D2O, 500 MHz): δ (in ppm) = 1.99 (m, 4H), 2.62 (t, 4H), 2.84 (t, 4H), 5.14 (d, 4H), 6.10 (m, 2H), 7.16 (d, 4H), 7.77 (dd, 4H), 8.24 (dd, 4H), 8.53 (d, 4H); ESI-MS: m/z calcd for C30H34N4 ²⁺ = 450.63 for [M²⁺- 2Br], found: 225 (m/2).

### Synthesis of the end piece substructure (8)

A 100 ml flask equipped with a magnetic stirrer was charged with 3.2 g trans-1,4-dibromo-2-butene (99%, 15 mmol, 2.1 equiv.) in 40 ml acetone (HPLC). Then, 1 ml triethylamine (0.72 g, 7.1 mmol, 1.0 equiv.) were added dropwise and the clear solution was stirred at 20 °C overnight. Thereby the solution became turbid. The precipitate was filtered off, washed with 20 ml acetone and dried in vacuum at 50 °C using a rotary evaporator. The compound **8** was obtained as a white solid (2.0 g, 89% yield). ¹H-NMR (DMSO-d6, 500 MHz): δ (in ppm) = 1.20 (t, 9H), 2.21 (q, 6H), 3.94 (d, 2H), 4.19 (d, 2H), 6.04 (m, 1H), 6.33 (m, 1H); ESI-MS: m/z calcd for C₁₀H₂₁BrN₃⁺ = 235.19 for [M⁺- Br-], found: 234, 236.

### Synthesis of compound (9)

A flask was charged with 600 mg compound **7** (1 mmol, 1.0 equiv.) and 650 mg compound **8** (2.1 mmol, 2.1 equiv.) in 25 ml methanol (HPLC) and the mixture was stirred for 15 hours at reflux temperature. Afterwards, the solvent was removed using a rotary evaporator and the residue was washed with diethyl ether (3 x 50 ml). The organic extracts were discarded and the residue was freeze-dried to give compound **9** as a yellowish foam (810 mg, 65% yield). ¹H-NMR (D₂O, 500 MHz): δ (in ppm) = 1.35 (t, 18H), 2.26 (m, 4H), 3.10 (m, 8H), 3.37 (q, 12H), 4.00 (d, 2H), 5.34 (d, 8H), 6.21 (m, 2H), 6.31 (q, 2H), 6.50 (m, 2H), 8.02 (t, 8H), 8.76 (t, 8H).

### Example 4

### Synthesis of polycationic compound (11)

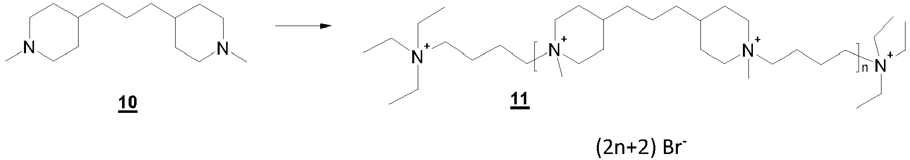

The starting material **10** was synthesized from commercially available 4,4'-trimethylene-dipiperidine according to the protocol described by A.P. Phillips in J. Amer. Chem. Soc., 1955, 76, 6396.

A 100 ml flask equipped with reflux condenser, magnetic stirrer and adsorber tube was charged with 1.0 g compound **10** (4.2 mmol, 1.0 equiv.) in 40 ml acetone (HPLC). To this solution, 0.35 ml triethylamine (0.26 g, 2.5 mmol, 0.6 equiv.) and 0.55 ml dibromobutane (1.0 g, 4.6 mmol, 1.1 equiv.) were added successively. The reaction mixture was stirred under reflux for 15 hours. Thereby a white precipitate was formed which was filtered off rapidly, washed with 50 ml acetone (HPLC) and dried under vacuum at 50 °C for 30 minutes. Compound **11** was obtained as a beige solid (1.87 g, 71% yield). ¹H-NMR (D₂O,
500 MHz): δ (in ppm) = 1.25 (CH₃ (TEA)), 1.3-1.4 (br, 6H), 1.5-1.7 (br, 6H), 1.7-1.8
(br, 2H), 1.8-2.0 (br, 6H), 2.42 (CH₂ (TEA)), 3.02 (s, 3H), 3.05 (s, 3H), 3.2-3.3 (m,
4H), 3.3-3.5 (m, 8H); M_{w} -59.000 g·mol⁻¹ according to the GPC method).

### Reference Example 5

### Synthesis of compound 13

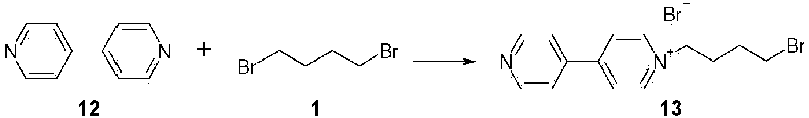

A 100 ml flask equipped with reflux condenser, magnetic stirrer and adsorber tube was charged with 1.0 g 4,4'-bipyridine (6.4 mmol, 1.0 equiv.) in 30 ml acetone (HPLC grade). To this solution 0.77 ml 1,4-dibromobutane (1.38 g, 6.4 mmol, 1.0 equiv.) were added in one portion. The reaction mixture was stirred under reflux for 15 hours. Thereby a precipitate formed, which was filtered off rapidly, washed twice with 50 ml acetone (HPLC grade) and dried under vacuum at 50 °C for 30 minutes. Compound **13** was obtained as a yellow solid (1.87 g, 79% yield). ¹H-NMR (D₂O, 500 MHz): δ (in ppm) = 1.97 (m, 2H), 2.23 (m, 2H), 3.55 (t, 2H), 4.73 (t, 2H), 7.91 (dd, 2H), 8.42 (d, 2H), 8.76 (dd, 2H), 9.00 (d, 2H); ESI-MS: m/z calcd for C₁₄H₁₆BrN₂ = 292.2 for [M⁺-Br⁻], found: 291; 293.

### Comparative Example 3

### Synthesis of dimeric compound 13a

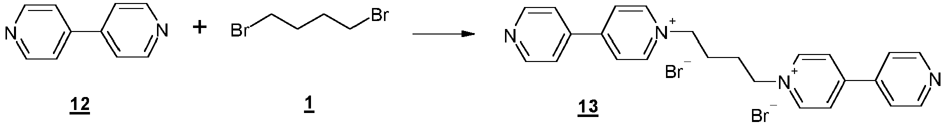

A 100 ml flask equipped with reflux condenser, magnetic stirrer and adsorber tube was charged with 1.0 g 4,4'-bipyridine (6.4 mmol, 1.0 equiv.) in 30 ml acetone (HPLC grade). To this solution, 0.68 ml 1,4-dibromobutane (1.23 g, 5.7 mmol, 0.89 equiv.)were added successively. The reaction mixture was stirred under reflux for 15 hours. Thereby a yellowish precipitate was formed which was filtered off rapidly, washed with 50 ml acetone (HPLC grade) and dried under vacuum at 50 °C for 30 minutes. The compound **13** was obtained as an orange solid (860 mg).

### Example 6.1

### Synthesis of compound 14a

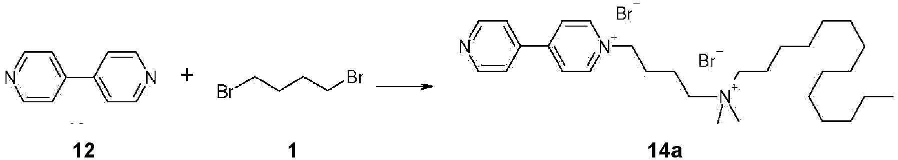

A 100 ml flask equipped with reflux condenser, magnetic stirrer and adsorber tube was charged with 1.0 g 4,4'-bipyridine (6.4 mmol, 1.0 equiv.) in 30 ml acetone (HPLC grade). To this solution, 0.84 ml 1,4-dibromobutane (1.52 g, 7.0 mmol, 1.1 equiv.) and 1.0 ml N,N-dimethyltetradecylamine (0.8 g, 3.3 mmol, 0.52 equiv.) were added successively. The reaction mixture was stirred under reflux for 15 hours. Thereby a yellowish precipitate formed which was filtered off rapidly, washed with 50 ml acetone (HPLC grade) and dried under vacuum at 50 °C for 30 minutes. The compound **14a** was obtained as an orange solid (2.46 g, 63%). ¹H-NMR (D₂O, 500 MHz): δ (in ppm) = 0.88 (t, 3H), 1.29-1.48 (m, 24H), 1.79 (m, 2H), 2.01 (m, 2H), 2.27 (s, 6H), 3.24 (t, 2H), 3.59 (t, 2H), 4.88 (t, 2H), 7.95 (m, 2H), 8.46 (dd, 2H), 8.81 (m, 2H), 9.02 (d, 2H).

### Example 6.2

### Synthesis of compound 14b

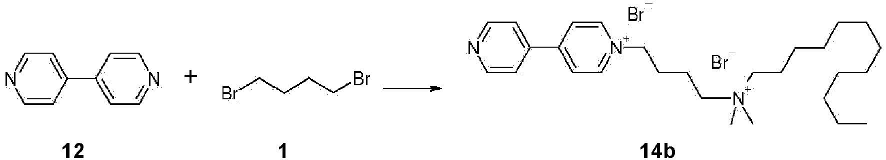

Compound **14b** was prepared according to the synthesis of 14a using 1.0 g 4,4'-bipyridine (6.4 mmol, 1.0 equiv.), 0.84 ml 1,4-dibromobutane (1.52 g, 7.0 mmol, 1.1 equiv.) and 0.85 ml N,N-dimethyldodecylamine (0.67 g, 3.15 mmol, 0.49 equiv.) in 30 ml acetone (HPLC grade) and was obtained as an orange solid (1.83 g, 49%). ¹H-NMR (D₂O, 500 MHz): δ (in ppm) = 0.88 (t, 3H), 1.1-1.4 (m, 20H), 1.77 (m, 2H), 2.01 (m, 2H), 2.27 (s, 6H), 3.11 (t, 2H), 3.59 (t, 2H), 4.87 (t, 2H), 7.95 (m, 2H), 8.47 (dd, 2H), 8.81 (m, 2H), 9.02 (d, 2H); ESI-MS: m/z calcd for C₂₈H₄₇N₃²⁺ = 425.7 for [M²⁺-2 Br⁻], found: 212 (m/2).

### Example 6.3

Compound 14c was prepared analogous to example 6.2 but using N,N-dimethyldecylamine in place of N,N-dimethyldodecylamine.

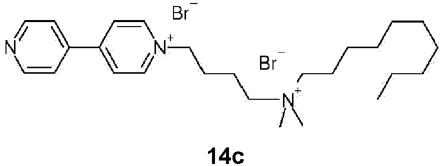

The reactions described above were also carried out using 1:1 methanol:dimethylformamide (v/v) as a solvent. Under said conditions, the products were freeze-dried to obtain the compounds as oils or foams.

The minimum inhibitory concentration (MIC) of the compounds of examples 1 to 5 was determined and is given in table 1 below. The following bacteria and fungi strains and methods were used:

Aspergillus brasiliensis (2d) and (9d): MIC determined in accordance with a method of the European Committee on Antimicrobial Susceptibility Testing: EUCAST E.DEF 9.1, July 2008, "Method for the determination of broth dilution minimum inhibitory concentrations of antifungal agents for conidia forming moulds" available under www.eucast.

Candida albicans: MIC determined in accordance with the method described in EUCAST DEFINITIVE DOCUMENT EDef 7.2 "Method for the determination of broth dilution minimum inhibitory concentrations of antifungal agents for yeasts" available under www.eucast.org.

Pseudomonas aeruginosa and Escherichia coli: MIC determined in accordance with DIN EN ISO 20776-1: 2006.

**Table 1:**

| **Microorganism** | **MIC (mg/l) of the compound of** | | | | | | | | |
|---|---|---|---|---|---|---|---|---|---|
| | Ex. 1.2 | Ex. 2 | Ex. 3 | Ex. 4 | Ref. Ex. 5 | Ex. 6.2 | Comp. ex. 1 | Comp. ex. 2 | Comp. ex. 3 |
| A. bras.¹⁾ (2d) | 6.3 - 12.5 | 1.6 - 6.3 | 12.5 | 3.1 | 3.1 | 6.3 | 1.6 | 1.56 | 200 |
| A. bras.¹⁾ (9d) | 6.3 - 12.5 | 6.3 | 12.5 - 25.0 | 3.1 - 6.3 | 3.1 - 50 | 6.3 | 12.5 | 3.12 | 200 - 400 |
| C. albicans²⁾ | 50 | <3.1 | <3.1 | <3.1 | 1.56 | <3.12 | 0.78 | <3.12 | 200 |
| P. aeruginosa³⁾ | 6.3 | 6.3 | 25 | 12.5 | 12.5 | 12.5 | 3.1 | 3.12 | >400 |
| E. coli⁴⁾ | 6.3 | 12.5 | 50 | 12.5 | 6.3 | 6.3 | 6.3 | 3.12 | 200 |

| | | | | | | | | | |
|---|---|---|---|---|---|---|---|---|---|
| ¹⁾ (DSM 1988/ ATCC 16404) ²⁾ (DSM 1386/ ATCC 10231) ³⁾ (DSM 1128/ ATCC 9027) ⁴⁾ (DSM 1576/ ATCC 8739) | | | | | | | | | |

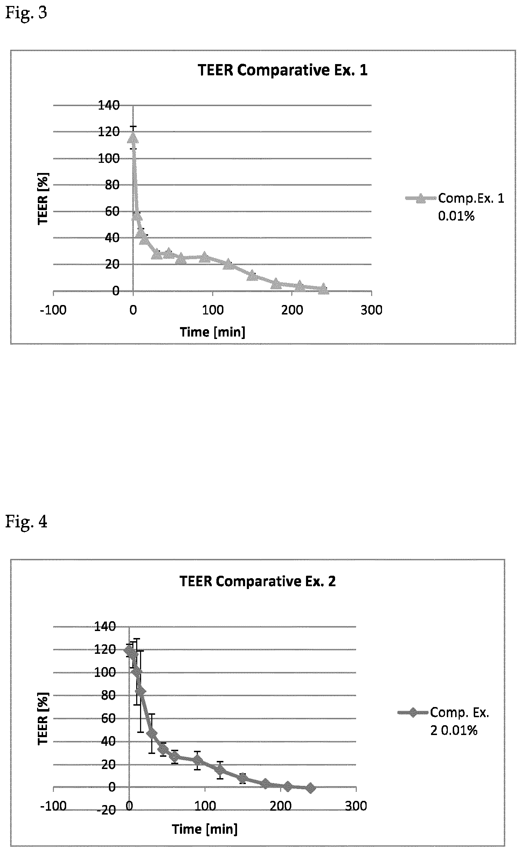

As can be seen, the compounds of the invention are highly active against fungi and bacteria, even against such problematic fungi like aspergillus brasiliensis and bacteria like pseudomonas aeruginosa. The compounds of comparative examples 1 and 2 have acceptable activity but are unsuitable for practical use because they Impair the cell barrier, see the TEER values below, figures 3 and 4.

The toxicity of the compounds of the invention was determined in accordance with a conventional MTT test. Human corneal epithelial cells (HCE-T-cell line) were cultivated in 5%Sasaki cell culture medium consisting of 250 ml DMEM (Dulbecco's Modified Eagle's Medium), 250 ml Ham's F12 medium, 27.17 ml fetale calf serum, 543.4 µl insulin 5 mg/ml, 1.086 ml EGF stock solution 5 µg/ml, 2.717 ml DMSO und 5.43 ml antibiotic/antimycotic solution. The cells were seeded into a 96 wells cell culture plate (18 000 cells/well) and grown in an incubator for about 24 h (37°C, 5% CO₂). The medium was removed and the test compounds dissolved in KRB (Krebs Ringer buffer) were applied. 100 µl KRB were used as positive control whereas a 0.5 % Triton X solution was used as negative control.

After incubation for 10 min. under light protection the test compounds were removed and the plates were rinsed once with 100 µl phosphate buffered saline (PBS without Mg and Ca) per well. After removal of PBS 100 µl MTT reagent (0.05%) were added into each well followed by incubation for 3h at 37 °C under light protection. Thereafter, the reagent was removed and replaced by 100 µl lysis solution. The dark blue dye was dissolved from the cells by shaking. After 30 min. the plate was evaluated by UV spectroscopy at 570 nm.

The results are given in table 2 below:

**Table 2:**

| **Compound¹⁾** | **Viability²⁾ (%)** |
|---|---|
| Ex. 1.2 | 86.8 ± 7.6 |
| Ex. 2 | 93.6 ± 11.1 |
| Ex. 3 | 90.3 ± 7.0 |
| Ex. 4 | 82.3 ± 9.1 |
| Ref. ex. 5 | 83.8 ± 12.6 |
| Ex. 6.2 | 74.9 ± 12.0 |
| Comp. ex. 1 | 83.0 ± 6.1 |
| Comp. ex. 2 | 87.0 ± 13.0 |

| | |
|---|---|
| ¹⁾ compound used in a concentration of 0.01 %. ²⁾ viability of human corneal epithelial cells measured after treatment with the test compound for 10 minutes in reference to KRB (Krebs-Ringer buffer, 100% viability) and Triton X (0% viability). | |

The table shows that the compounds of the invention exhibit low toxicity levels and are therefore highly compatible.

### TEER values (TEER: transepithelial electrical resistance)

TEER is a method to determine the barrier strength of epithelial cells. By determining the barrier strength the effect of a test substance on epithelial cells may be evaluated. TEER values were determined as follows:

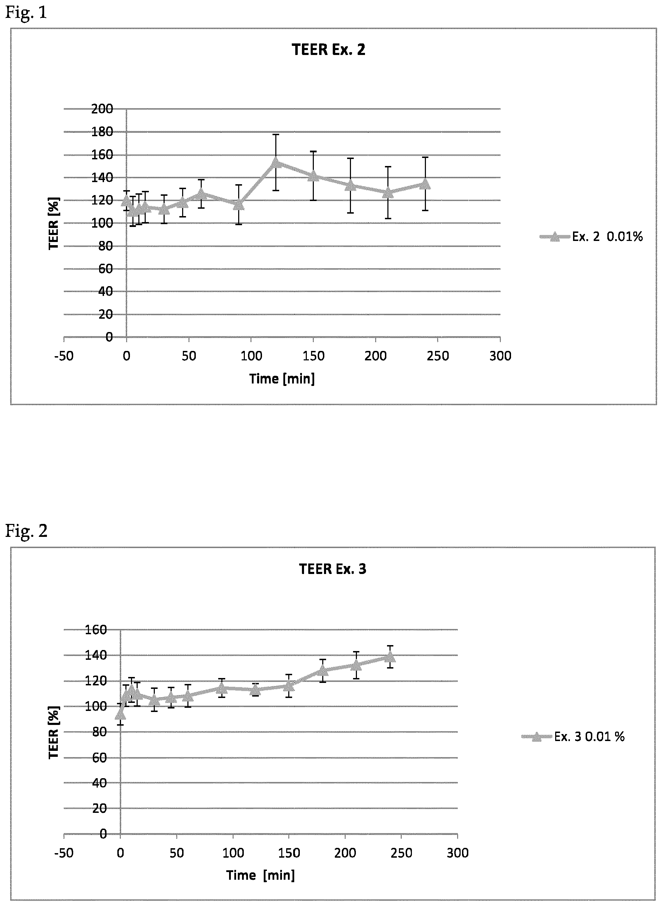

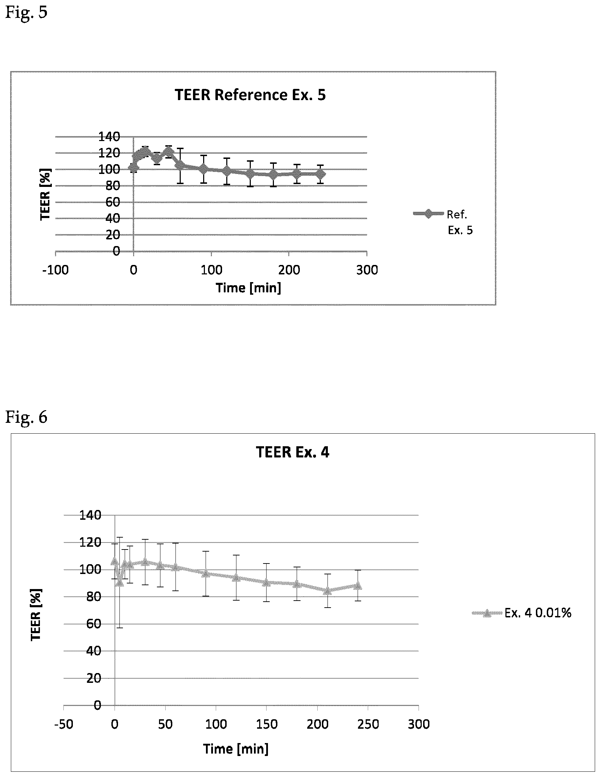

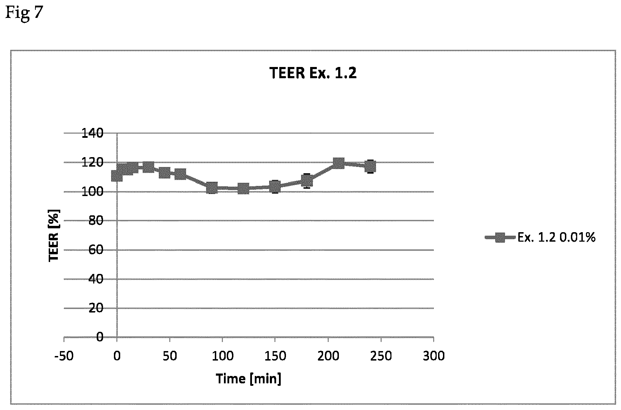

The test was performed with the MDCK-1 cell line using a ThinCert™ cell culture insert in a 24 well format (Greiner Bio-one International GmbH) with 100.000 cells being charged into each well. Underneath the ThinCert™ 1.5 ml and onto the ThinCert™ 0.6 ml cell culture medium were given (MDCK-1 medium containing 500 ml MEM, 10% fetal calf serum, 2mM L-glutamine and 1% antibiotic). The cells were seeded on day 1 and then incubatedat 37 °C and 5% CO₂. On day 3 and 4 the cell culture medium was changed. On day 5 the TEER values were determined using an STX electrode and an Evom measuring device. The initial TEER values were determined (time 0). Thereafter, the cell culture medium was removed from the ThinCerts™ and replaced by prewarmed KRB of pH 7.4 (6.8 g NaCl, 0.4 g KCI, 0.14 g NaH₂PO4 x H₂O, 2.1 g NaHCO₃, 3.575 g HEPES, 1.1 D-glucose monohydrate, 0.2 g MgSO₄ x 7 H₂O, 0.26 g CaCl₂ x 2 H₂O, aqua bidest. ad 1000 ml). After 30 min the TEER values were again determined. Subsequently, the test substances dissolved in KRB (0.01 % and 0.1 %) were placed on the upper side of the ThinCerts™. The TEER values were then determined at intervals during 4 hours (5 min, 10 min, 15 min, 30 min, 45 min, 60 min, 1 h 30 min, 2 h, 2 h 30 min, 3 h, 3 h 30 min, 4 h). ThinCerts™ where only KRB was added to the cell layers were used as control. The compounds were tested in a concentration of 0.01%. The results for the compounds of examples 1.2, 2, 3, 4, 6.2, 6.3, and comparative examples 1, 2 and 5 are shown in figures 1 to 7. As can be seen, the TEER values of the compounds of the invention decrease only to a small extent, in particular during the first 90 minutes. This means that the compounds of the invention do not essentially impair the cell barrier so that the cells remain intact and their permeability for impurities etc. is not reduced. This is an essential contribution to the safety of the compounds.

## Claims

1. Cationic compounds of formula (I) 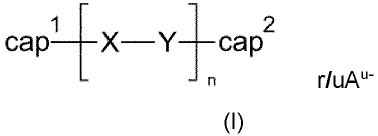 wherein
a)
X is 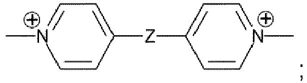
Y is C₄-C₆ alkenylene;
Z is C₁-C₆ alkylene;
cap¹ is 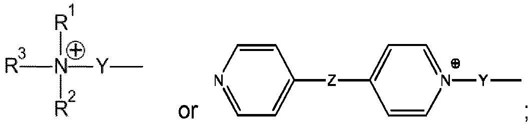
cap² is 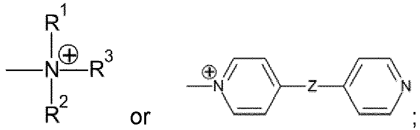
R¹, R² and R³ are independently of each other C₁-C₆ alkyl or C₁-C₆ hydroxyalkyl or one of R¹, R² and R³ is C₈-C₁₈ alkyl and the other two are independently of each other C₁-C₆ alkyl or C₁-C₆ hydroxyalkyl; and
n is 1 to 250;
b)
X is 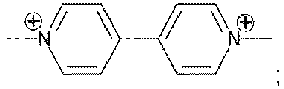 Y is C₂-C₆ alkylene;
cap¹ is 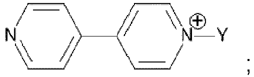
cap² is 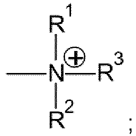
R¹ and R² are independently of each other C₁-C₆ alkyl;
R³ is C₈-C₁₈ alkyl; and
n is 0 to 2;
c)
X is 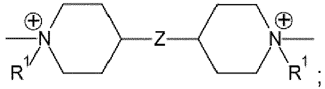
Y is C₂-C₆ alkylene;
Z is C₁-C₆ alkylene which is different from Y;
cap¹ is 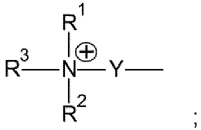
cap² is 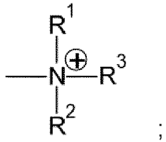
R¹, R² and R³ are independently of each other C₁-C₆ alkyl; and
n is 5 to 200, preferably 30 to 150;
d)
X is 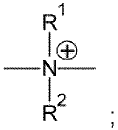
Y is C₂-C₆ alkylene;
cap¹ is 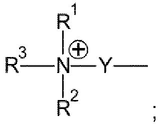
cap² is 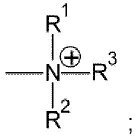
R¹ and R² are independently of each other C₁-C₆ alkyl;
R³ is C₁₂-C₁₆ alkyl; and
n is 100 to 400;
A is an anion;
u is the valency of the anion; and
r is the number of positive charges in the compound.

2. A cationic compound of claim 1, corresponding to the compounds of group (a) having the formula (Ia): 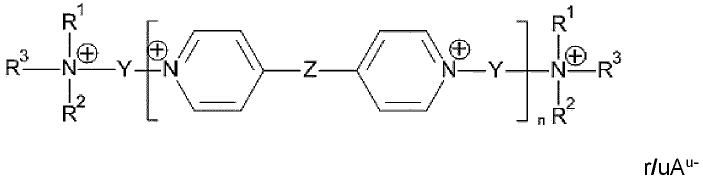 wherein n, r, u, A, Y, Z, R¹, R² and R³ are as defined in claim 1.

3. A cationic compound of claim 2, wherein R¹, R² and R³ are methyl or ethyl, Y is C₄-alkenylene, Z is C₂-C₄ alkylene, and n is 2 to 15.

4. A cationic compound of claim 2 or 3, wherein n is 2.

5. A cationic compound of claim 1 corresponding to the compounds of group (b) having the formula (Ib): 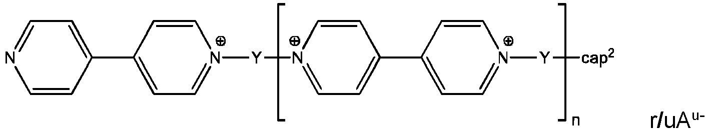 wherein n, r, u, A, Y and cap² are as defined in claim 1.

6. A cationic compound of claim 5, wherein R¹ and R² are methyl, R³ is C₁₀-C₁₆ alkyl and Y is C₂-C₄ alkylene.

7. A cationic compound of claim 6, wherein n is 0.

8. A cationic compound of claim 1 corresponding to the compounds of group (c) having the formula (Ic): 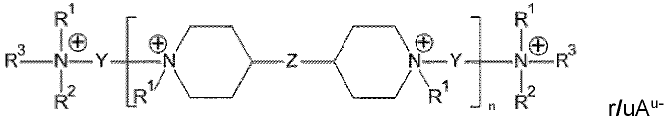 wherein n, r, u, A, Y, R¹, R² and R³ are as defined in claim 1.

9. A cationic compound of claim 8, wherein R¹, R² and R³ are methyl, Z is C₁-C₃ alkylene, Y is C₄-C₆ alkylene and n is 10 to 30.

10. A cationic compound of claim 1, corresponding to the compounds of group (d) having the formula (Id): 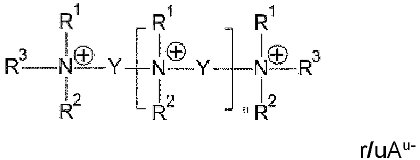 wherein n, r, u, A, Y, R¹, R² and R³ are as defined in claim 1.

11. A cationic compound of claim 10, wherein R¹ and R² are methyl, R³ is C₁₂-C₁₆ alkyl, and Y is C₃-C₄ alkylene.

12. The cationic compounds of claim 1 selected from 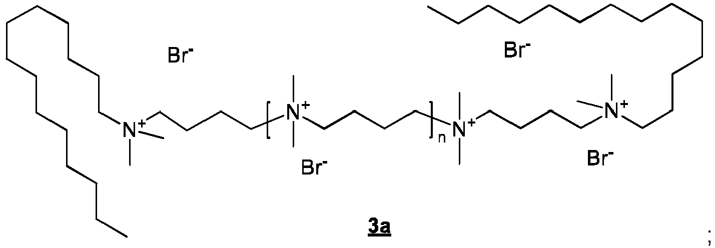 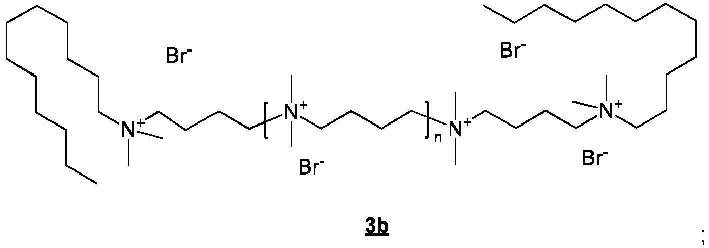 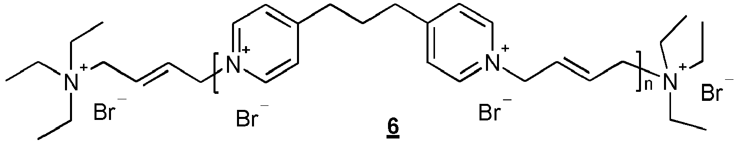 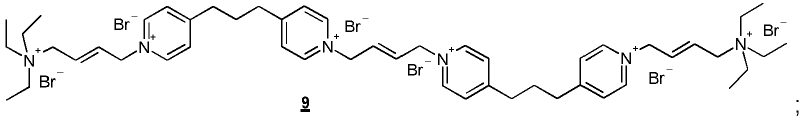 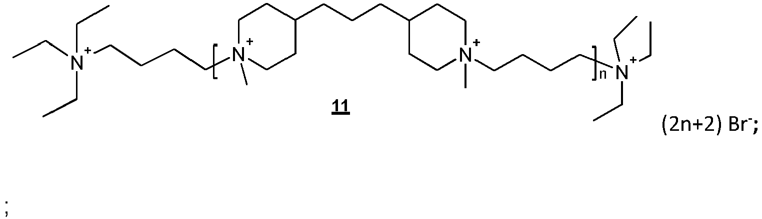 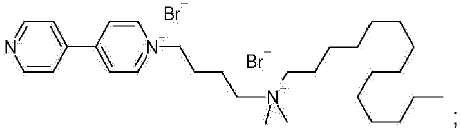 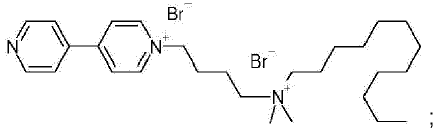 and

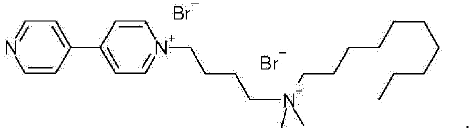

13. A composition comprising a cationic compound of any one of claims 1 to 12.

14. A composition of claim 13 in the form of a disinfectant formulation for medical devices or in the form of a preservative formulation for liquid, semi-solid or solid drugs, ophthalmic formulations or cosmetics.

15. A composition of claim 13 in the form of an ophthalmic composition.

16. A composition of any one of claims 13 to 15 which comprises 0.0001 to 1.0 w/v% of the cationic compound.

17. A composition of any one of claims 13 to 16 which comprises an additional disinfectant or preservative.

18. The use of a cationic compound of formula (I') as a disinfectant or preservative 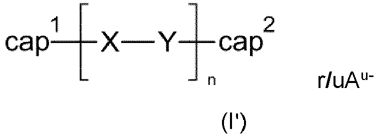 wherein
a)
X is 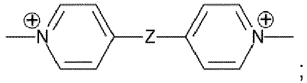
Y is C₄-C₆ alkenylene;
Z is C₁-C₆ alkylene;
cap¹ is 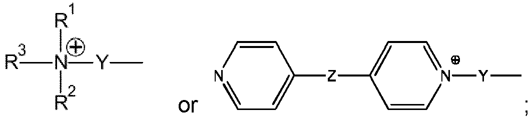
cap² is 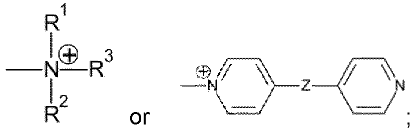
R¹, R² and R³ are independently of each other C₁-C₆ alkyl or C₁-C₆ hydroxyalkyl or one of R¹, R² and R³ is C₈-C₁₈ alkyl and the other two are independently of each other C₁-C₆ alkyl or C₁-C₆ hydroxyalkyl; and
n is 1 to 250;
b)
X is 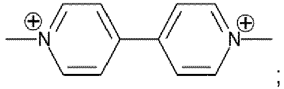
Y is C₂-C₆ alkylene;
cap¹ is 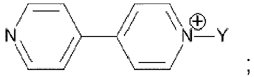
cap² is 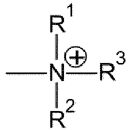 or a halogen atom;
R¹ and R² are independently of each other C₁-C₆ alkyl;
R³ is C₈-C₁₈ alkyl; and
n is 0 to 2;
c)
X is 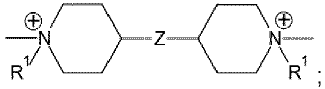
Y is C₂-C₆ alkylene;
Z is C₁-C₆ alkylene which is different from Y;
cap¹ is 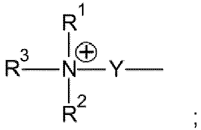
cap² is 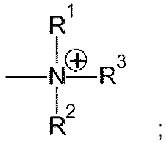
R¹, R² and R³ are independently of each other C₁-C₆ alkyl; and
n is 5 to 200, preferably 30 to 150;
d)
X is 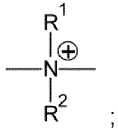
Y is C₂-C₆ alkylene;
cap¹ is 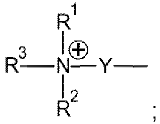
cap² is 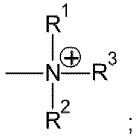
R¹ and R² are independently of each other C₁-C₆ alkyl;
R³ is C₁₂-C₁₆ alkyl; and
n is 100 to 400;
A is an anion;
u is the valency of the anion; and
r is the number of positive charges in the compound.

19. The use of a cationic compound of any one of claims 2 to 12 or of a composition of any one of claims 13 to 17 as a disinfectant or preservative.

## Patentansprüche

1. Kationische Verbindungen der Formel (I) 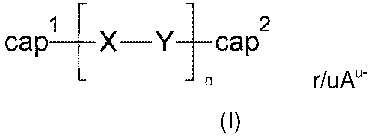 worin
a)
X für 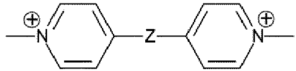 steht;
Y für C₄-C₆ Alkenylen steht;
Z für C₁-C₆ Alkylen steht;
cap¹ für 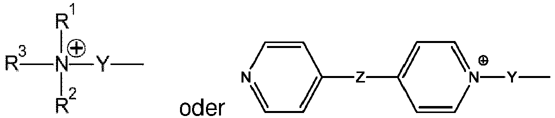 steht;
cap² für 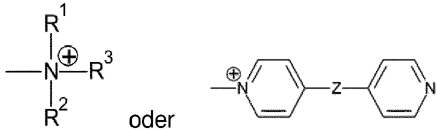 steht;
R¹, R² und R³ unabhängig voneinander für C₁-C₆ Alkyl oder C₁-C₆ Hydroxyalkyl oder einer von R¹, R² und R³ für C₈-C₁₈ Alkyl und die anderen beiden unabhängig voneinander für C₁-C₆ Alkyl oder C₁-C₆ Hydroxyalkyl stehen; und
n für 1 bis 250 steht;
b)
X für 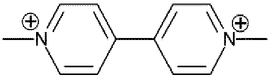 steht;
Y für C₂-C₆ Alkylen steht;
cap¹ für 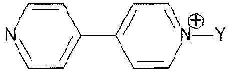 steht;
cap² für 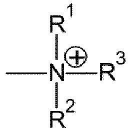 steht;
R¹ und R² unabhängig voneinander für C₁-C₆ Alkyl stehen; R³ für C₈-C₁₈ Alkyl steht; und
n für 0 bis 2 steht;
c)
X für 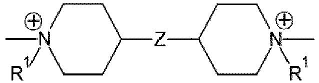 steht;
Y für C₂-C₆ Alkylen steht;
Z für ein von Y verschiedenes C₁-C₆ Alkylen steht;
cap¹ für 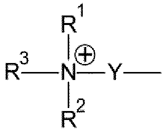 steht;
cap² für 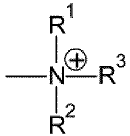 steht;
R¹, R² und R³ unabhängig voneinander für C₁-C₆ Alkyl stehen; und
n für 5 bis 200, bevorzugt 30 bis 150 steht;
d)
X für 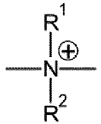 steht;
Y für C₂-C₆ Alkylen steht;
cap¹ für 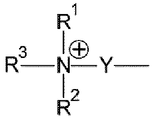 steht;
cap² für 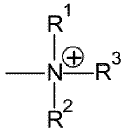 steht;
R¹ und R² unabhängig voneinander für C₁-C₆ Alkyl stehen;
R³ für C₁₂-C₁₆ Alkyl steht; und
n für 100 bis 400 steht;
A für ein Anion steht;
u für die Valenz des Anions steht; und
r für die Anzahl an positiven Ladungen in der Verbindung steht.

2. Kationische Verbindung nach Anspruch 1, entsprechend den Verbindungen der Gruppe (a) mit der Formel (la): 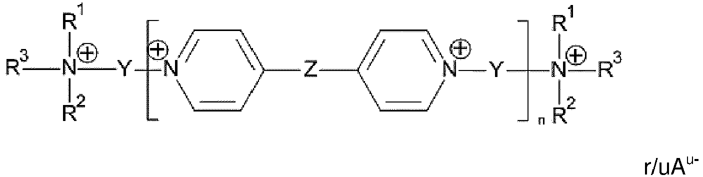 worin n, r, u, A, Y, Z, R¹, R² und R³ wie in Anspruch 1 definiert sind.

3. Kationische Verbindung nach Anspruch 2, worin R¹, R² und R³ für Methyl oder Ethyl, Y für C₄-Alkenylen, Z für C₂-C₄ Alkylen, und n für 2 bis 15 steht.

4. Kationische Verbindung nach Anspruch 2 oder 3, worin n für 2 steht.

5. Kationische Verbindung nach Anspruch 1, entsprechend den Verbindungen der Gruppe (b) mit der Formel (Ib): 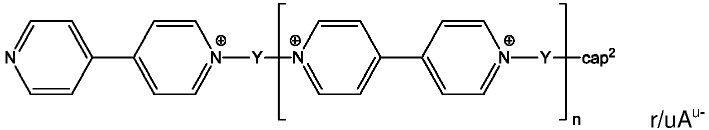 worin n, r, u, A, Y und cap² wie in Anspruch 1 definiert sind.

6. Kationische Verbindung nach Anspruch 5, worin R¹ und R² für Methyl, R³ für C₁₀-C₁₆ Alkyl und Y für C₂-C₄ Alkylen steht.

7. Kationische Verbindung nach Anspruch 6, worin n für 0 steht.

8. Kationische Verbindung nach Anspruch 1, entsprechend den Verbindungen der Gruppe (c) mit der Formel (Ic): 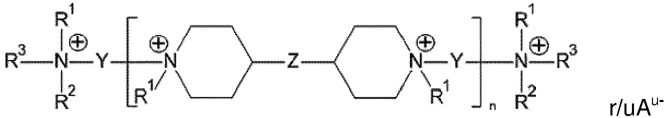 worin n, r, u, A, Y, R¹, R² und R³ wie in Anspruch 1 definiert sind.

9. Kationische Verbindung nach Anspruch 8, worin R¹, R² und R³ für Methyl, Z für C₁-C₃ Alkylen, Y für C₄-C₆ Alkylen und n für 10 bis 30 steht.

10. Kationische Verbindung nach Anspruch 1, entsprechend den Verbindungen der Gruppe (d) mit der Formel (Id): 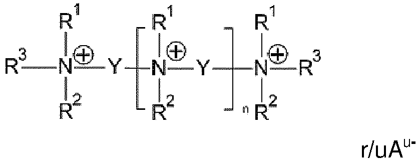 worin n, r, u, A, Y, R¹, R² und R³ wie in Anspruch 1 definiert sind.

11. Kationische Verbindung nach Anspruch 10, worin R¹ und R² für Methyl, R³ für C₁₂-C₁₆ Alkyl, und Y für C₃-C₄ Alkylen steht.

12. Die kationischen Verbindungen nach Anspruch 1, ausgewählt unter 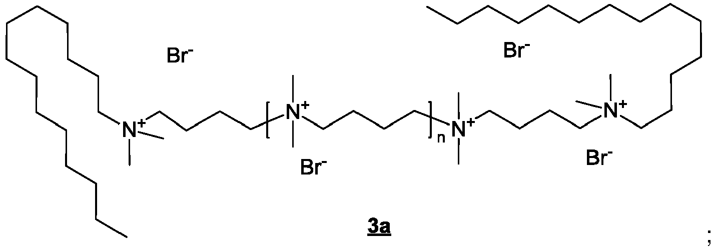 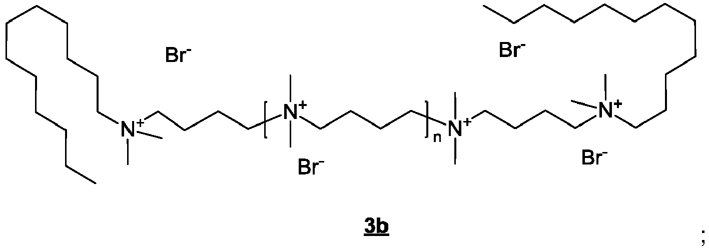 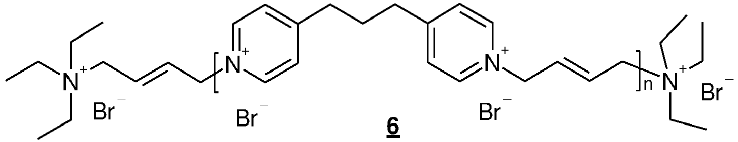 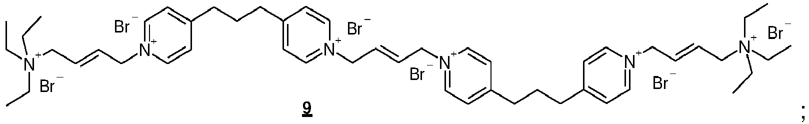 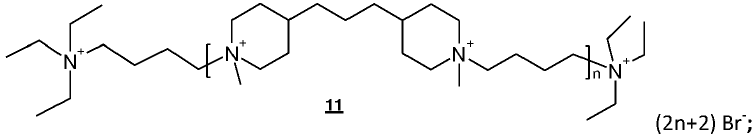 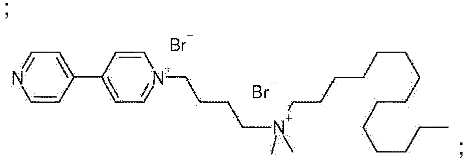 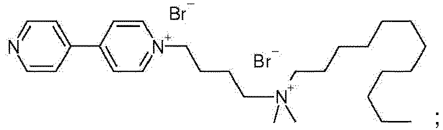 und

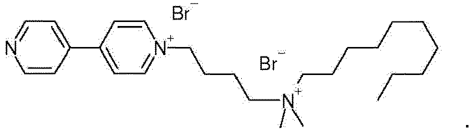

13. Zusammensetzung, umfassend eine kationische Verbindung nach einem der Ansprüche 1 bis 12.

14. Zusammensetzung nach Anspruch 13 in Form einer Desinfektionsmittelzusammensetzung für medizinische Geräte oder in Form einer Konservierungsmittelzusammensetzung für flüssige, halbfeste oder feste Arzneimittel, ophthalmische Zusammensetzungen oder Kosmetika.

15. Zusammensetzung nach Anspruch 13 in Form einer ophthalmischen Zusammensetzung.

16. Zusammensetzung nach einem der Ansprüche 13 bis 15, die 0,0001 bis 1,0 w/v% der kationischen Verbindung umfasst.

17. Zusammensetzung nach einem der Ansprüche 13 bis 16, die ein zusätzliches Desinfektionsmittel oder Konservierungsmittel umfasst.

18. Verwendung einer kationischen Verbindung nach Formel (I') als Desinfektionsmittel oder Konservierungsmittel 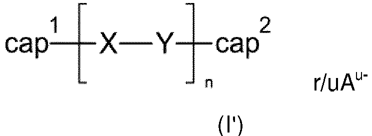 worin
a)
X für 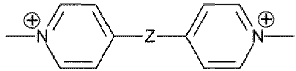 steht;
Y für C₄-C₆ Alkenylen steht;
Z für C₁-C₆ Alkylen steht;
cap¹ für 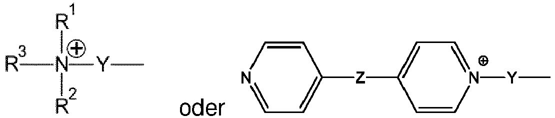 steht;
cap² für 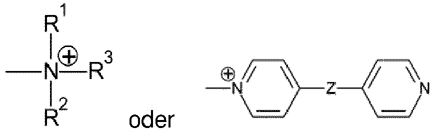 steht;
R¹, R² und R³ unabhängig voneinander für C₁-C₆ Alkyl oder C₁-C₆ Hydroxyalkyl oder einer von R¹, R² und R³ für C₈-C₁₈ Alkyl und die anderen beiden unabhängig voneinander für C₁-C₆ Alkyl oder C₁-C₆ Hydroxyalkyl stehen; und
n für 1 bis 250 steht;
b)
X für 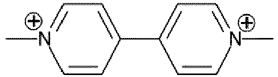 steht;
Y für C₂-C₆ Alkylen steht;
cap¹ für 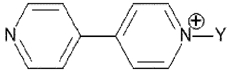 steht;
cap² für 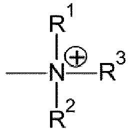 oder ein Halogenatom steht;
R¹ und R² unabhängig voneinander für C₁-C₆ Alkyl stehen;
R³ für C₈-C₁₈ Alkyl steht; und
n für 0 bis 2 steht;
c)
X für 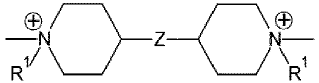 steht;
Y für C₂-C₆ Alkylen steht;
Z für ein von Y verschiedenes C₁-C₆ Alkylen steht;
cap¹ für 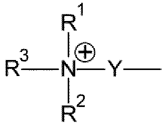 steht;
cap² für 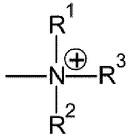 steht;
R¹, R² und R³ unabhängig voneinander für C₁-C₆ Alkyl stehen; und
n für 5 bis 200, bevorzugt 30 bis 150 steht;
d)
X für 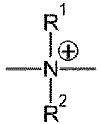 steht;
Y für C₂-C₆ Alkylen steht;
cap¹ für 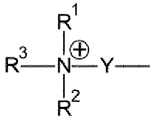 steht;
cap² für 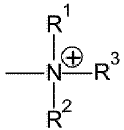 steht;
R¹ und R² unabhängig voneinander für C₁-C₆ Alkyl stehen;
R³ für C₁₂-C₁₆ Alkyl steht; und
n für 100 bis 400 steht;
A für ein Anion steht;
u für die Valenz des Anions steht; und
r für die Anzahl an positiven Ladungen in der Verbindung steht.

19. Verwendung einer kationischen Verbindung nach einem der Ansprüche 2 bis 12 oder einer Zusammensetzung nach einem der Ansprüche 13 bis 17 als Desinfektionsmittel oder Konservierungsmittel.

## Revendications

1. Composés cationiques de formule (I) 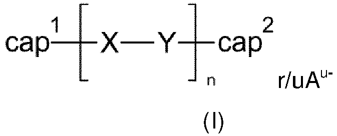 dans lesquels
a)
X est 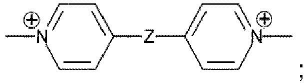
Y est alcénylène en C₄-C₆ ;
Z est alkylène en C₁-C₆;
cap¹ est 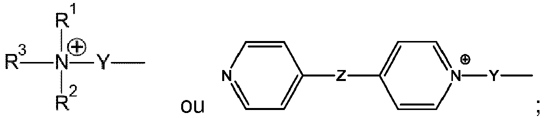
cap² est 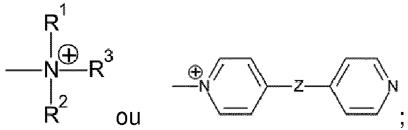
R¹, R² et R³ sont indépendamment les uns des autres alkyle en C₁-C₆ ou hydroxyalkyle en C₁-C₆ ou l'un de R¹, R² et R³ est alkyle en C₈-C₁₈ et les deux autres sont indépendamment l'un de l'autre alkyle en C₁-C₆ ou hydroxyalkyle en C₁-C₆; et
n est 1 à 250;
b)
X est 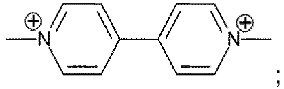
Y est alkylène en C₂-C₆;
cap¹ est 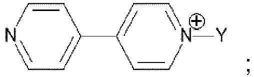
cap² est 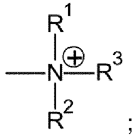
R¹ et R² sont indépendamment l'un de l'autre alkyle en C₁-C₆ ;
R³ est alkyle en C₈-C₁₈ ; et
n est 0 à 2 ;
c)
X est 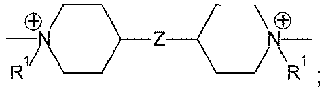
Y est alkylène en C₂-C₆ ;
Z est alkylène en C₁-C₆ qui est différent de Y ;
cap¹ est 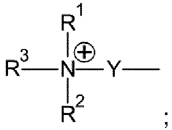
cap² est 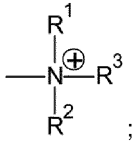
R¹, R² et R³ sont indépendamment les uns des autres alkyle en C₁-C₆ ; et
n est 5 à 200, de préférence 30 à 150 ;
d)
X est 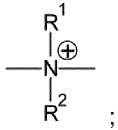
Y est alkylène en C₂-C₆;
cap¹ est 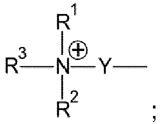
cap² est 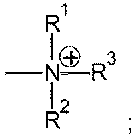
R¹ et R² sont indépendamment l'un de l'autre alkyle en C₁-C₆ ;
R³ est alkyle en C₁₂-C₁₆ ; et
n est 100 à 400;
A est un anion ;
u est la valence de l'anion ; et
r est le nombre de charges positives dans le composé.

2. Composé cationique selon la revendication 1, correspondant aux composés du groupe (a) ayant la formule (Ia) : 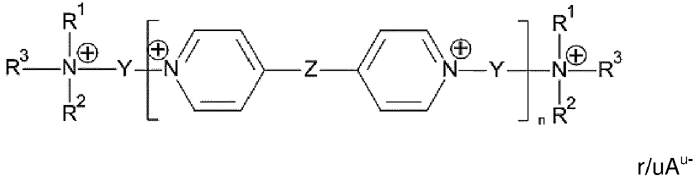 dans lequel n, r, u, A, Y, Z, R¹, R² et R³ sont tels que définis dans la revendication 1.

3. Composé cationique selon la revendication 2, dans lequel R¹, R² et R³ sont méthyle ou éthyle, Y est alcénylène en C₄, Z est alkylène en C₂-C₄ et n est compris entre 2 et 15.

4. Composé cationique selon la revendication 2 ou 3, dans lequel n vaut 2.

5. Composé cationique selon la revendication 1 correspondant aux composés du groupe (b) ayant la formule (Ib) : 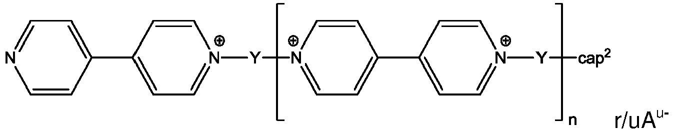 dans lequel n, r, u, A, Y et cap² sont tels que définis dans la revendication 1.

6. Composé cationique selon la revendication 5, dans lequel R¹ et R² sont méthyle, R³ est alkyle en C₁₀-C₁₆ et Y est alkylène en C₂-C₄.

7. Composé cationique selon la revendication 6, dans lequel n vaut 0.

8. Composé cationique selon la revendication 1 correspondant aux composés du groupe (c) ayant la formule (Ic) : 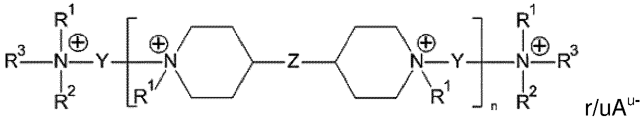 dans lequel n, r, u, A, Y, R¹, R² et R³ sont tels que définis dans la revendication 1.

9. Composé cationique selon la revendication 8, dans lequel R¹, R² et R³ sont méthyle, Z est alkylène en C₁-C₃, Y est alkylène en C₄-C₆ et n est de 10 à 30.

10. Composé cationique selon la revendication 1, correspondant aux composés du groupe (d) ayant la formule (Id) : 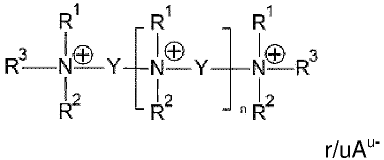 dans lequel n, r, u, A, Y, R¹, R² et R³ sont tels que définis dans la revendication 1.

11. Composé cationique selon la revendication 10, dans lequel R¹ et R² sont méthyle, R³ est alkyle en C₁₂-C₁₆ et Y est alkylène en C₃-C₄.

12. Composés cationiques selon la revendication 1 choisis parmi 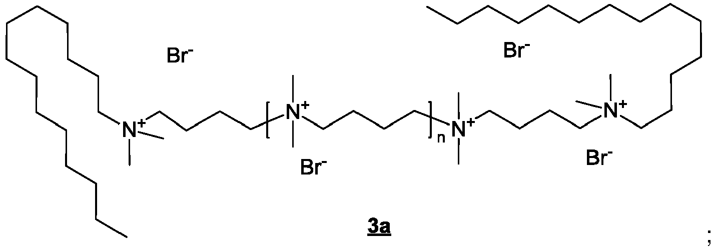 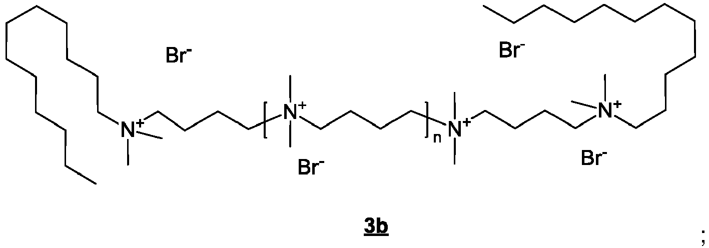 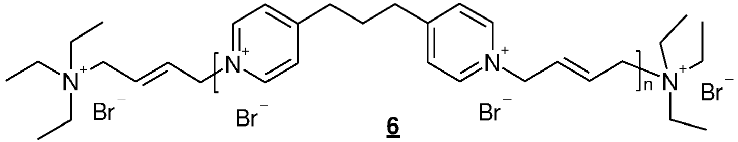 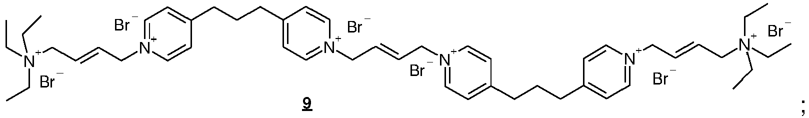 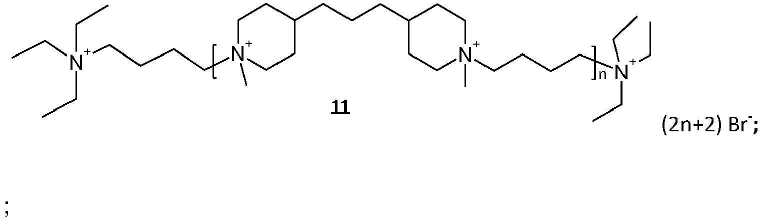 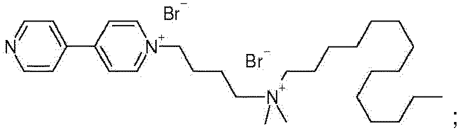 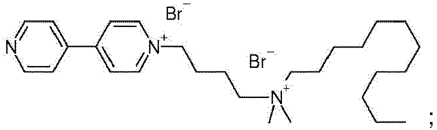 et

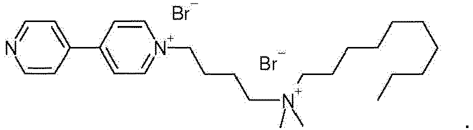

13. Composition comprenant un composé cationique selon l'une quelconque des revendications 1 à 12.

14. Composition selon la revendication 13 sous forme de formulation désinfectante pour dispositifs médicaux ou sous forme de formulation conservatrice pour médicaments liquides, semi-solides ou solides, formulations ophtalmiques ou produits cosmétiques.

15. Composition selon la revendication 13 sous la forme d'une composition ophtalmique.

16. Composition selon l'une quelconque des revendications 13 à 15, qui comprend 0,0001 à 1,0 % en poids/volume du composé cationique.

17. Composition selon l'une quelconque des revendications 13 à 16, qui comprend un désinfectant ou un conservateur supplémentaire.

18. Utilisation d'un composé cationique de formule (I') comme désinfectant ou conservateur 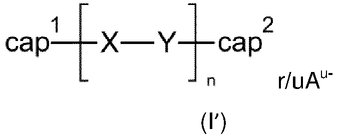 dans laquelle
a)
X est 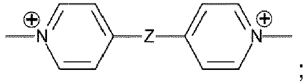
Y est alcénylène en C₄-C₆ ;
Z est alkylène en C₁-C₆;
cap¹ est 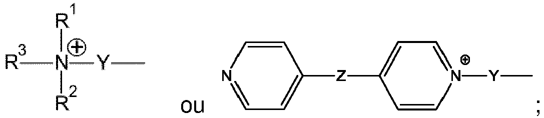
cap² est 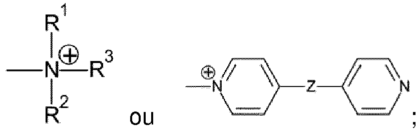
R¹, R² et R³ sont indépendamment les uns des autres alkyle en C₁-C₆ ou hydroxyalkyle en C₁-C₆ ou l'un de R¹, R² et R³ est alkyle en C₈-C₁₈ et les deux autres sont indépendamment l'un de l'autre alkyle en C₁-C₆ ou hydroxyalkyle en C₁-C₆ ; et
n est 1 à 250;
b)
X est 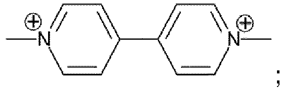 Y est alkylène en C₂-C₆ ;
cap¹ est 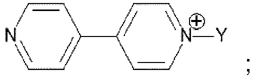
cap² est 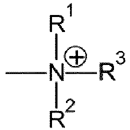 ou un atome d'halogène ;
R¹ et R² sont indépendamment l'un de l'autre alkyle en C₁-C₆ ;
R³ est alkyle en C₈-C₁₈ ; est
n est 0 à 2;
c)
X est 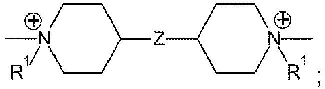
Y est alkylène en C₂-C₆ ;
Z est alkylène en C₁-C₆ qui est différent de Y;
cap¹ est 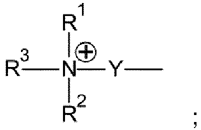
cap² est 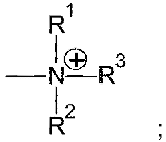
R¹, R² et R³ sont indépendamment les uns des autres alkyle en C₁-C₆ ; et n est 5 à 200, de préférence 30 à 150 ;
d)
X est 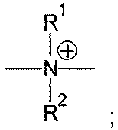
Y est alkylène en C₂-C₆ ;
cap¹ est 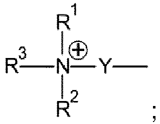
cap² est 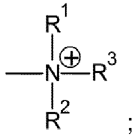 R¹ et R² sont indépendamment l'un de l'autre alkyle en C₁-C₆ ;
R³ est alkyle en C₁₂-C₁₆ ; et
n est 100 à 400;
A est un anion ;
u est la valence de l'anion ; et
r est le nombre de charges positives dans le composé.

19. Utilisation d'un composé cationique selon l'une quelconque des revendications 2 à 12 ou d'une composition selon l'une quelconque des revendications 13 à 17 en tant que désinfectant ou conservateur.
